# EUROPÄISCHE PATENTSCHRIFT

(11) **EP 2 465 332 B1**
(45) Veröffentlichungstag und Bekanntmachung des Hinweises auf die Patenterteilung: **20.11.2019**
(21) Anmeldenummer: 10754701.0
(22) Anmeldetag: 08.08.2010
(51) Int. Cl.: H05H 1/24

(54) **VORRICHTUNG ZUR BEHANDLUNG VON LEBENDEN ZELLEN MITTELS EINES PLASMAS**
DEVICE FOR TREATING LIVING CELLS BY MEANS OF A PLASMA
DISPOSITIF DESTINÉ AU TRAITEMENT DE CELLULES VIVANTES AU MOYEN D'UN PLASMA

(30) Priorität: 11.08.2009 DE 102009028462
(43) Veröffentlichungstag der Anmeldung: 20.06.2012
(73) Patentinhaber: Leibniz-Institut für Plasmaforschung und Technologie e.V., 17489 Greifswald (DE)
(72) Erfinder: WELTMANN, Klaus-Dieter, 18609 Binz (DE); VON WOEDTKE, Thomas, 18519 Sundhagen (DE); LADEMANN, Olaf, 18055 Rostock (DE)
(74) Vertreter: Schulz Junghans Patentanwälte PartGmbB
(86) Internationale Anmeldenummer: PCT/EP2010/061528
(87) Internationale Veröffentlichungsnummer: WO 2011/018423

(56) Entgegenhaltungen:
- EP-A1- 1 765 044
- EP-A1- 2 160 081
- WO-A1-2009/060213
- US-A1- 2007 029 500
- WANG S ET AL: "Discharge comparison of nonequilibrium atmospheric pressure Ar/O2 and He/O2 plasma jets", APPLIED PHYSICS LETTERS, AIP USA, Bd. 83, Nr. 16, 20. Oktober 2003 (2003-10-20), Seiten 3272-3274, XP002617380, ISSN: 0003-6951
- FRIDMAN G ET AL: "Applied plasma medicine", PLASMA PROCESSES AND POLYMERS, Bd. 5, Nr. 6, August 2008 (2008-08), Seiten 503-533, XP002617381, WILEY-VCH VERLAG GMBH GERMANY ISSN: 1612-8850

## Beschreibung

Die Erfindung betrifft eine Vorrichtung zur Behandlung lebender Zellen mittels eines Plasmas (Plasmaporation) und eine Verwendung der Vorrichtung. Die Vorrichtung enthält neben herkömmlichen Einrichtungen zur Plasmaerzeugung, Einrichtungen zur Mischung und zum Transport von Wirkstoffen, die vorwiegend in Form von verkapselten Nano- und Mikropartikeln, die von der Plasmaentladung nicht zerstört werden, vorliegen.

### [Stand der Technik]

### 1. Problem der Krebstherapie:

Die einzeln oder in Kombination verwendeten Standardverfahren der Krebstherapie sind heute die Operation, die Chemotherapie und die Strahlungstherapie. In der klinischen Erprobung befindet sich derzeit eine Methode, die als "Elektroporationstherapie" (EPT), "Elektrochemotherapie" (ECT) bzw. "Hochspannungs-Impulstherapie" (HVIT) bezeichnet wird und darauf basiert, dass durch die gezielte Anwendung gepulster elektrischer Felder selektiv die Membranporen der Krebszellen kurzzeitig reversibel geöffnet werden (Elektroporation), so dass eine drastisch erhöhte Aufnahme der für die Chemotherapie verwendeten Wirkstoffe, wie beispielsweise Bleomycin bzw. Cisplatin durch die Zellen erzielt wird. Die Vorteile gegenüber der konventionellen Chemotherapie bestehen darin, dass bei der Elektrochemotherapie die erforderliche Wirkstoffdosis etwa zwanzigfach niedriger gewählt werden kann, wegen der selektiven Abtötung eine Gewebe schonende Wirkung bei minimaler Narbenbildung und eine starke Reduzierung der durch das Chemotherapeutikum hervorgerufenen Nebenwirkungen erreicht wird. Da sich die Krebszellen von den gesunden Zellen hinsichtlich ihrer Größe, Struktur der Zellmembran und der elektrischen Eigenschaften unterscheiden, kann eine Selektivität der Elektroporation durch eine geeignete Wahl der Amplitude, Anzahl, Dauer und Frequenz der Hochspannungsimpulse erreicht werden. In den Patentschriften DE69604509, DE69928383 und DE60106901 werden verschiedene Vorrichtungen dafür beschrieben, eine solche gesteuerte, selektive Elektroporation von Zellen zu realisieren.

US2007 0029500 A1 offenbart eine Plasmavorrichtung zur Hautbehandlung und Ablagerung von fluoreszierenden Partikeln.

Auch wenn bei der Elektrochemotherapie die Wirkstoffdosis beträchtlich reduziert werden kann, so kann doch nicht völlig auf den Einsatz teurer Chemotherapeutika verzichtet werden, die, zwar in geringerem Maße als bei konventioneller Chemotherapie, Nebenwirkungen auf gesundes Körpergewebe haben.

### 2. Problem der Heilung chronischer Wunden:

Da chronische Wunden meist die Folge von Grunderkrankungen sind, besteht der primäre Ansatz für ihre Heilung in der Diagnostik und Kausaltherapie dieses Grundleidens. In vielen Fällen ist die Heilung der Grunderkrankung jedoch nicht möglich, so dass durch vielfältige lokaltherapeutische Maßnahmen, wie beispielsweise durch chirurgische Eingriffe, Wundreinigung und -desinfektion, durch den Einsatz von Antibiotika sowie von speziellen Wundauflagen und Wundverbänden, versucht wird, eine Heilung der Wunde trotz fortbestehender Grunderkrankung zu erreichen.

Das ständig zunehmende Angebot an Therapeutika (Salben, Tinkturen, Puder, Antiseptika, Antibiotika) sowie Verbandmaterialien und Verbandstoffsystemen zur Behandlung chronischer Wunden, und das damit verbundene Problem der richtigen Auswahl und Anwendung, führt zu einem therapeutischen Vorgehen, das durch eine große Zahl verschiedener, unkoordinierter und oft wirkungsloser ärztlicher Maßnahmen gekennzeichnet ist (-Polypragmasie) und oft die Ursache für Heilungsverzögerungen und Kostensteigerungen ist.

### 3. Problem bei der topischen Applikation von Wirkstoffen in die Haut:

Die Haut ist nicht nur das größte Organ unseres Organismus, sie stellt auch die Barriere unseres Körpers zur Umwelt dar. Sie verhindert den Verlust von Wasser und den Eintritt von Umweltschadstoffen in unsere Haut. Diese Barrierefunktion stellt ein großes Problem bei der topischen Applikation von Wirkstoffen dar, d.h. von Substanzen, die auf die Haut aufgetragen werden und die im Bereich der lebenden Zellen wirken sollen. Hierfür ist es notwendig, dass diese Substanzen das Stratum Corneum durchdringen.

Vielfältige Methoden wurden entwickelt, um diesen Prozess zu stimulieren. Hierzu gehört die Verwendung von verschiedenen Formulierungen, welche die Penetration von topisch applizierten Substanzen unterstützen. Dazu gehören Mikroemulsionen und Liposome. Neben der Einflussnahme über die Formulierung gibt es auch eine ganze Reihe von Methoden, die die Barriere künstlich schädigen, um ein verbessertes Eindringen von Wirkstoffen zu ermöglichen. Hierzu gehört die lontophorese sowie der Einsatz von Mikronadeln, welche kleine, ca. 100-200 µm tiefe Löcher in der Haut erzeugen und damit eine verbesserte Penetration ermöglichen. Darüber hinaus werden auch verschiedene Arten von Okklusionen eingesetzt, um topisch applizierte Substanzen in den Bereich der lebenden Zellen zu bringen.

### [Aufgabe der Erfindung]

Der Erfindung lag die Aufgabe zugrunde, die Nachteile der im Stand der Technik beschriebenen Lösungen zu beseitigen.

### Lösung der Aufgabe

Die Aufgabe wurde gemäß den Merkmalen der Patentansprüche gelöst.

Der Erfindung liegt die Idee zugrunde, auf lebende Zellen mittels eines Plasmas Substanzen einwirken zu lassen. Im Rahmen von Untersuchungen zum Einsatz eines Plasmajets zur Behandlung der Hautoberfläche, zur Desinfektion, wurde festgestellt, dass topisch applizierte Farbstoffe bei der Plasmabehandlung die Barriere der Haut sehr effektiv und schnell durchdringen können. Das ist sowohl möglich, wenn diese topisch applizierten Substanzen vor der Plasmabehandlung aber auch nach der Plasmabehandlung aufgetragen werden.

Überraschenderweise kommt es durch die Wechselwirkung zwischen Plasma und Gewebe zu einer Änderung der Zusammensetzung der Lipidstrukturen, so dass faktisch kurzzeitig eine "Tür" aufgeht, die einen Eintritt der topisch applizierten Substanzen in den Bereich der lebenden Zellen ermöglicht. Wie lange diese "Tür" offen bleibt, hängt von der eingebrachten Energie und der Temperatur des Plasmas ab. Unter günstigen Bedingungen ist es auch möglich, die topisch applizierten Substanzen nach der Plasmabehandlung auf die Haut aufzutragen und trotzdem eine effiziente und effektive Penetration im Bereich der lebenden Zellen zu erreichen. Nach diesem Zeitfenster bilden sich die Lipidstrukturen im Bereich der Hornschicht wieder in ihre ursprüngliche Form zurück und ein weiteres Eindringen von topisch applizierten Substanzen ist damit nicht mehr möglich. Dieses hat gleichzeitig den Vorteil, dass durch die Plasmabehandlung nur die gezielt eingesetzten topisch applizierten Substanzen die Barriere durchdringen, aber es nicht zu einem langzeitigen Öffnen der Barriere kommt, so dass Viren, Bakterien und Umweltschadstoffe diese Eintrittspforte nicht nutzen können, um in den Bereich der lebenden Zellen zu gelangen.

Die Untersuchungen mit In-vivo-Laser-Scan-Mikroskopie haben diesen Prozess bestätigt. Klar konnte gezeigt werden, dass ohne Plasmabehandlung eine fluoreszenzmarkierte Formulierung, die topisch appliziert wurde, nur im Bereich der Hautoberfläche bzw. in den oberen Schichten des Stratum Corneums nachgewiesen werden konnte. Nach der Plasmabehandlung konnte diese Formulierung auch im Bereich der lebenden Zellen mit hoher Konzentration nachgewiesen werden.

Auf der Grundlage dieser Erfindung ist es möglich, folgende Probleme zu lösen:
1. Problem der Krebstherapie: lokale, selektive Abtötung von Krebszellen, Applikation von Wirkstoffen, z.B. von Chemotherapeutika in geringer Dosis
2. Problem der Heilung chronischer Wunden: neuer therapeutischer Ansatz zur Verbesserung der Wundbehandlung als Kombination einer antiseptischen Wirkung mit einer Stimulierung der Neubildung von gesundem Gewebe und durch gezielte Applikation von Wirkstoffen
3. Problem bei der topischen Applikation von Wirkstoffen in die Haut: Durchdringung der oberen Schichten des Stratum Corneums.

Überraschenderweise ist es gelungen, Substanzen so in die Haut einzubringen, dass diese in der Plasmaentladung nicht zerstört oder umgewandelt werden. Ein entscheidender Lösungsansatz dabei ist die Stabilisierung von chemischen Substanzen gegenüber der elektrischen Entladung (hierbei sind verschiedene chemische Verfahren denkbar), nämlich eine Verkapselung der Wirkstoffe in Nano- oder Mikropartikel, die eine Hülle besitzen, die von der Plasmaentladung nicht zerstört wird. Diese Nano- oder Mikropartikel werden in die Haut eingeschleust und dort (im Bereich der lebenden Zellen) löst sich die Hülle auf und der Wirkstoff wird freigesetzt.

Im Fall der Behandlung der Zellen von chronischen Wunden kann durch die parallele Anwendung von Elektroporation und Atmosphärendruckplasma bei optimaler Wahl der Behandlungsparameter eine wirksame antiseptische Wirkung des Plasmas bei gleichzeitiger Stimulierung der Neubildung von gesunden Gewebezellen erzielt werden.

Gleichzeitig wird eine Sterilisation dadurch erreicht, dass durch eine reversible Elektroporation bei gleichzeitiger Plasmawirkung die Zellmembranporen der abzutötenden Mikroorganismen temporär geöffnet werden und dadurch eine effektivere Abtötung der Zellen durch die im Plasma erzeugten Radikale erfolgen kann.

Im Fall einer Anwendung zur Krebstherapie wird durch die Reduzierung der Dosis teurer Chemotherapeutika eine beträchtliche Senkung der Behandlungskosten erreicht.

Bei der Behandlung von chronischen Wunden besteht der Vorteil der Erfindung in der Möglichkeit, von einer polypragmatischen Behandlung zu einer effizienten und kostengünstigeren, universell anwendbaren Behandlung zu gelangen, durch die eine effektive antiseptische Wirkung erzielt wird, die mit der Stimulierung einer Neubildung von gesundem Gewebe verbunden ist.

Durch die zusätzliche Anwendung einer Elektroporation während der Sterilisation, Dekontamination oder Aufbereitung medizintechnischer Produkte mittels Atmosphärendruckplasmen wird eine wesentliche Verbesserung der Sterilisationseffizienz erzielt.

Die erfindungsgemäße Vorrichtung zur Behandlung von lebenden Zellen mittels eines Plasmas ist dadurch gekennzeichnet, dass sie neben herkömmlichen Einrichtungen zur Plasmaerzeugung und Erzeugung eines elektrischen Feldes, vorzugsweise mittels Elektroporation, Einrichtungen zur Mischung, zum Transport und zur Verkapselung von Wirkstoffen, vorzugsweise in Form von Nano- und Mikropartikeln zum Einbringen in Zellen und Gewebe, enthält. Bei dem Plasma handelt es sich vorzugsweise um ein Normaldruckplasma, insbesondere um ein kaltes oder nicht-thermisches Atmosphärendruckplasma. Die erfindungsgemäße Vorrichtung umfasst mindestens eine Elektrode, mindestens eine Gaszufuhr, mindestens eine Gasmischeinrichtung oder Mischeinrichtung von Gasen mit Mikro- und Nanopartikeln und mindestens eine Spannungsversorgung. Zusätzlich besitzt die Vorrichtung eine Gasdüse, mindestens ein Dielektrikum, Isolation und mindestens eine Elektrode (Niederspannung bis Hochspannung) sowie mindestens einen Gaskanal zur Mischung oder zum Transport von Wirkstoffen im Gasstrom.

Das nicht beanspruchte Verfahren umfasst die folgenden Schritte, wobei diese Schritte nacheinander oder gleichzeitig stattfinden können:
◆ Mischen von Gasen mit Wirkstoffen, vorzugsweise in Form von Mikro- und Nanopartikeln
◆ Anlegen eines Plasmas
◆ ggf. Erzeugung eines elektrischen Feldes, vorzugsweise mittels Elektroporation
◆ Applikation der Wirkstoffe mittels des Plasmas und ggf. des elektrischen Feldes unter die oberen Schichten des Stratum Corneums der Haut

Die Wirkstoffe können gegenüber der elektrischen Entladung stabilisiert werden, vorzugsweise durch eine Verkapselung (funktionelle Oberfläche).

Die erfindungsgemäße Verwendung der beschriebenen Vorrichtung ist in Anspruch 6 offenbart.

Beschrieben ist auch ein nicht beanspruchtes Verfahren zur Behandlung von Hauterkrankungen, Krebserkrankungen, chronischen Wunden, zur Unterstützung der postoperativen Wundheilung, Erkrankungen des Zahnfleisches (z.B. Parodontitis) und der Knochenheilung, gekennzeichnet dadurch, dass das Plasma zusätzlich Wirkstoffe im Gasstrom der Entladung transportiert.

Ebenfalls beschrieben ist auch ein nicht beanspruchtes Verfahren zum Einbringen von Wirkstoffen in Zellen zur Beeinflussung des Stoffwechsels der Zellen, wobei die Zellen durch die Plasmaporation selektiv zur Einbringung von Wirkstoffen (chemischen Substanzen) geöffnet werden.

Der Umfang der Erfindung ist durch die Ansprüche definiert.

Mit der vorgestellten Erfindung wird erstmals die Behandlung lebender Zellen mittels eines kalten, nicht-thermischen Atmosphärendruckplasmas bei gleichzeitiger Elektroporation der Zellen und gleichzeitiger oder zeitnaher Einbringung von Wirkstoffen in Form von Mikro- und Nanopartikeln zur Beeinflussung des Stoffwechsels der Zellen ermöglicht. Der Wirkstoff wird mit dem Gasstrom des Plasmajets auf die Haut aufgebracht und durch die durch das Plasma geöffnete Barriere in den Bereich der lebenden Zellen transportiert wird. Nach Ende des Plasma-Gewebe-Kontaktes wird die Barriere sofort wieder verschlossen. Es bilden sich intakte Strukturen heraus, die ein Eindringen von Bakterien, Viren und Pilzen in den Bereich der lebenden Haut verhindern.

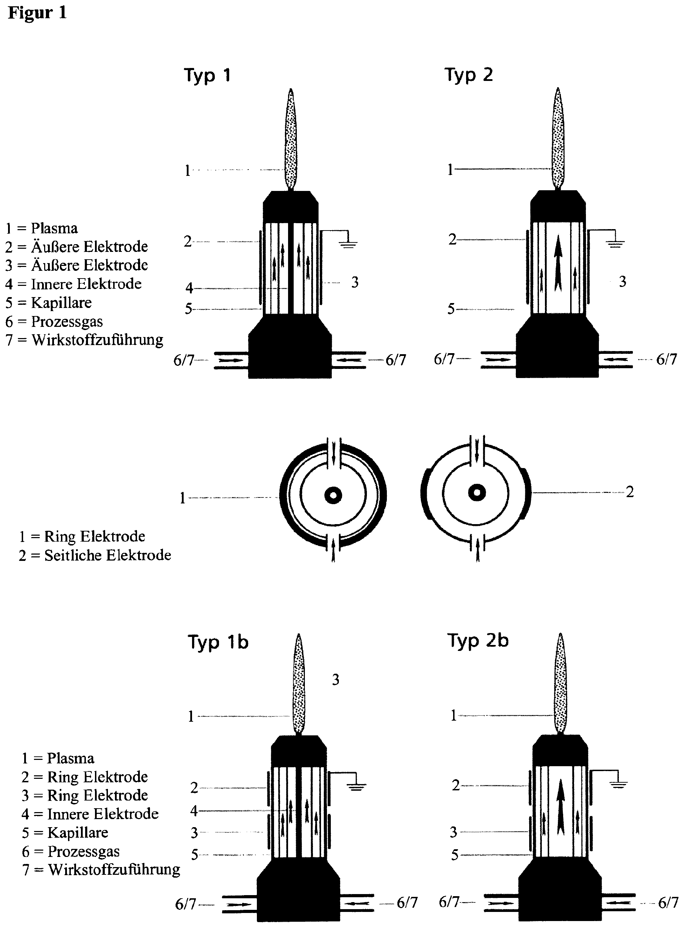

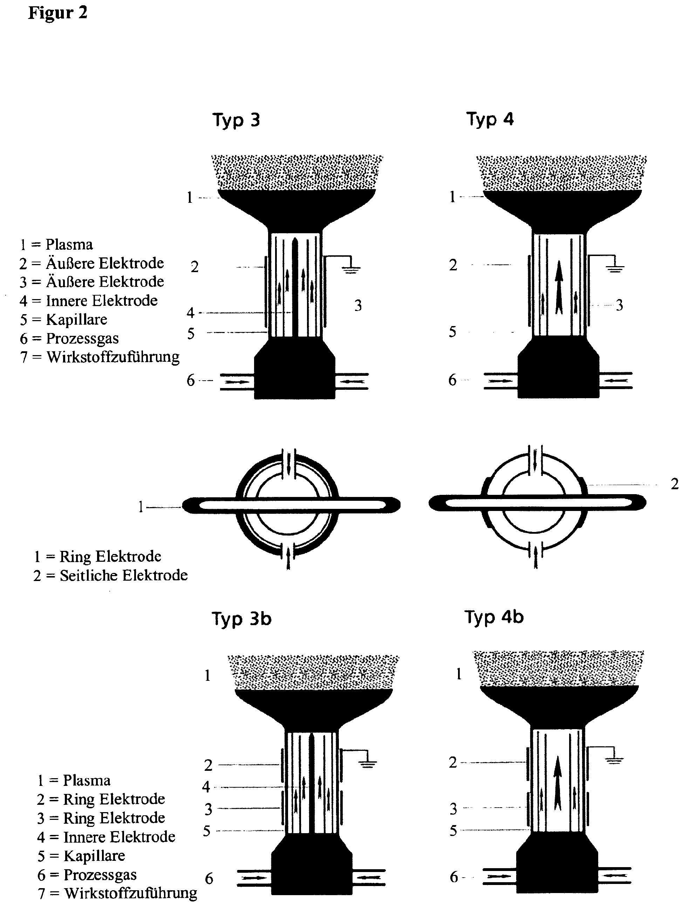

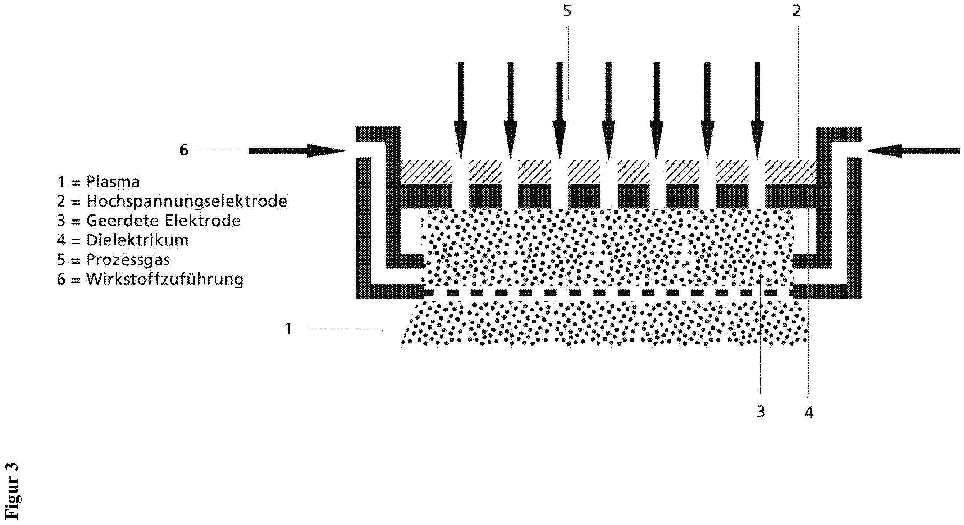

Die Erfindung wird anhand von Zeichnungen näher erläutert, ohne sie auf diese Zeichnungen zu beschränken. Die Fig. 1 bis Fig. 3 zeigen den prinzipiellen Aufbau der erfindungsgemäßen Vorrichtung.

## Patentansprüche

1. Vorrichtung zur Behandlung von lebenden Zellen mittels eines Plasmas, wobei die Vorrichtung herkömmliche Einrichtungen zur Erzeugung eines Plasmas aufweist, **dadurch gekennzeichnet, dass** die Vorrichtung weiterhin Einrichtungen zur Mischung von verkapselten Wirkstoffen in Form von Nano- und Mikropartikeln, die eine Hülle besitzen, die von der Plasmaentladung nicht zerstört wird, und zum Transport dieser verkapselten Wirkstoffe in einen Gasstrom der Plasma-Entladung aufweist.

2. Vorrichtung nach Anspruch 1, **dadurch gekennzeichnet, dass** es sich um ein Normaldruckplasma handelt.

3. Vorrichtung nach einem der Ansprüche 1 bis 2, **dadurch gekennzeichnet, dass** die Vorrichtung herkömmliche Einrichtungen zur Erzeugung eines elektrischen Feldes aufweist.

4. Vorrichtung nach einem der Ansprüche 1 bis 3, **dadurch gekennzeichnet, dass** sie mindestens eine Elektrode, mindestens eine Gaszufuhr, mindestens eine Gasmischeinrichtung oder Mischeinrichtung von Gasen mit Wirkstoffen und mindestens eine Spannungsversorgung enthält.

5. Vorrichtung nach Anspruch 4, **dadurch gekennzeichnet, dass** sie zusätzlich eine Gasdüse, mindestens ein Dielektrikum, Isolation und mindestens eine Elektrode sowie mindestens einen Gaskanal zur Mischung oder zum Transport der Wirkstoffe im Gasstrom enthält.

6. Verwendung der Vorrichtung nach einem der Ansprüche 1 bis 5 zur Einbringung und Aufbringung von Wirkstoffen in Zellen oder auf die Oberfläche von Gegenständen, pflanzlichem und tierischem Gut.

## Claims

1. An apparatus for treating living cells by means of a plasma, wherein the apparatus has conventional devices for generating a plasma, **characterized in that** the apparatus furthermore has devices for mixing encapsulated active ingredients in the form of nanoparticles and microparticles which possess a sheath which is not destroyed by the plasma discharge, and for transporting said encapsulated active ingredients into a gas stream of the plasma discharge.

2. The apparatus according to Claim 1, **characterized in that** the plasma is an atmospheric-pressure plasma.

3. The apparatus according to any one of Claims 1 to 2, **characterized in that** the apparatus has conventional devices for generating an electric field.

4. The apparatus according to any one of Claims 1 to 3, **characterized in that** it contains at least one electrode, at least one gas supply, at least one gas mixing device or device for mixing gases and active ingredients and at least one voltage supply.

5. The apparatus according to Claim 4, **characterized in that** it additionally contains a gas nozzle, at least one dielectric, insulation and at least one electrode as well as at least one gas channel for mixing or for transporting the active ingredients in the gas stream.

6. Use of the apparatus according to any one of Claims 1 to 5 for introducing active ingredients into cells or applying active ingredients to the surface of objects and plant-based and animal products.

## Revendications

1. Dispositif destiné au traitement de cellules vivantes au moyen d'un plasma, dans lequel le dispositif présente des appareils traditionnels servant à générer un plasma, **caractérisé en ce que** le dispositif présente encore des appareils servant à mélanger des substances actives encapsulées sous la forme de nanoparticules et de microparticules, qui possèdent une enveloppe, qui n'est pas détruite par la décharge plasmique, et à transporter ces substances actives encapsulées dans un flux gazeux de la décharge plasmique.

2. Dispositif selon la revendication 1, **caractérisé en ce qu'**il s'agit d'un plasma généré sous une pression normale.

3. Dispositif selon l'une des revendications 1 à 2, **caractérisé en ce que** le dispositif présente des appareils traditionnels servant à générer un champ électrique.

4. Dispositif selon l'une des revendications 1 à 3, **caractérisé en ce qu'**il comporte au moins une électrode, au moins une alimentation en gaz, au moins un appareil de mélange gazeux ou un appareil pour mélanger des gaz avec des substances actives et au moins une alimentation électrique.

5. Dispositif selon la revendication 4, **caractérisé en ce qu'**il comprend en plus une buse à gaz, au moins un diélectrique, une isolation et au moins une électrode, ainsi qu'au moins un conduit de gaz servant à mélanger ou à transporter les substances actives dans le flux gazeux.

6. Utilisation du dispositif selon l'une des revendications 1 à 5 pour introduire et appliquer des substances actives dans des cellules ou à la surface d'objets, de biens d'origine animale et végétale.
